(19) Europäisches Patentamt
European Patent Office
Office européen des brevets

(11) EP 2 348 100 B1

(12) **EUROPEAN PATENT SPECIFICATION**

(45) Date of publication and mention of the grant of the patent:
**07.09.2016 Bulletin 2016/36**

(21) Application number: **09827332.9**

(22) Date of filing: **17.11.2009**

(51) Int Cl.:
***C12N 1/16*** *(2006.01)* ***C12N 1/18*** *(2006.01)*
***C12P 13/04*** *(2006.01)*

(86) International application number:
**PCT/JP2009/006148**

(87) International publication number:
**WO 2010/058551 (27.05.2010 Gazette 2010/21)**

(54) **METHOD FOR PRODUCING AMINO-ACID-RICH YEAST**

VERFAHREN ZUR HERSTELLUNG AMINOSÄUREREICHER HEFE

PROCÉDÉ POUR LA PRODUCTION DE LEVURE RICHE EN ACIDE AMINÉ

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**RS**

(30) Priority: **18.11.2008 JP 2008294644**

(43) Date of publication of application:
**27.07.2011 Bulletin 2011/30**

(60) Divisional application:
**15175951.1 / 2 949 745**

(73) Proprietor: **Asahi Group Holdings, Ltd.**
**Tokyo 130-8602 (JP)**

(72) Inventors:
- **SHIBUYA, Ichiro**
  **Moriya-shi**
  **Ibaraki 302-0106 (JP)**
- **ODANI, Tetsuji**
  **Moriya-shi**
  **Ibaraki 302-0106 (JP)**

(74) Representative: **Desaix, Anne et al**
**Ernest Gutmann - Yves Plasseraud S.A.S.**
**3, rue Auber**
**75009 Paris (FR)**

(56) References cited:
**EP-A1- 0 805 202**
**EP-A2- 0 143 560**
**GB-A- 1 196 391**
**JP-A- 9 294 581**
**JP-A- 9 313 169**
**JP-A- 10 327 802**
**JP-A- 60 133 892**
**JP-A- 63 123 390**
**JP-A- 2002 171 961**
**JP-A- 2006 129 835**
**JP-A- 2007 049 989**
**JP-B- 39 016 342**
**JP-B- 54 031 076**
**JP-B2- 3 896 606**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 348 100 B1

## Description

[Technical Field]

The present invention relates to a method for producing an amino acid-rich yeast.

[Background Art]

Currently, mainly in advanced countries such as Europe, USA, and Japan, natural- and health-conscious natural seasonings which do not use additives are required all over the world. Under such the circumstances, in the yeast extract industry, high-value added extracts having enhanced "deliciousness (umami)" of nucleic acids have been developed, and also the development of amino acids as a representative of "deliciousness" along with nucleic acids has advanced.

As for glutamic acid, sodium glutamate has hitherto been spread as a chemical seasoning. However, in recent years, use of a culture or extract obtained by culturing a yeast containing naturally not only glutamic acid but also other amino acids in a food and drink is favored.

For example, Patent Literature 1 describes a yeast extract characterized in that the amount of a free amino acid is 25% by weight or more, and also the total amount of nucleic acid-based taste-active components is 2% by weight or more.

Also, Patent Literature 2 describes a yeast extract composition derived from an yeast belonging to the genus Candida tropicalis, Candida lipolytica or Candida utilis, in which the total amount of free amino acid in a yeast extract is 3.0% or more, the amount of alanine in the total amount of free amino acid is 10% or more, the amount of glutamic acid is 25% or more, and the amount of histidine is 10% or more.

Also, Patent Literature 3 describes a sweetness improving agent containing a yeast extract as an active component, and the yeast extract contains sodium 5'-inosinate and/or sodium 5'- adenylate, sodium 5'-guanylate, sodium 5'- uridylate and sodium 5'- cytidylatem respectively in a proportion of 1 to 15%, and sodium glutamate in a proportion of 1 to 20%.

Also, Patent Literature 4 describes a method for producing a yeast extract containing intracellular free glutamine-derived glutaminic acid in a proportion of at least 3% relative to extract solid parts, the method including the step of digesting a yeast containing free glutamine in an amount of 15 mg or more per 1 g of dry cells.

Also, Patent Literature 5 describes a yeast extract which is a yeast extract obtained by digesting or decomposing a yeast, in which a peptide fraction having a molecular amount of 10,000 or more accounts for 10% or more based on the total amount of the entire peptides, said peptides being detected by absorptiometry at 220 nm in an effluent prepared by passing a sample solution containing the yeast extract through a filter membrane having a pore diameter of 1 micrometer, followed by gel filtration of the portion passed through the filter membrane.

Also, Patent Literature 6 describes a glutaminic acid-rich yeast extract containing 13% by weight or more of L-glutaminic acid (as a Na salt).

Also, Patent Literature 7 describes a seasoning composition containing a nucleic acid-based taste-active component, glutaminic acids, potassium and lactic acid, sodium lactate or potassium lactate, wherein the molar ratio of nucleic acid-based taste-active component:glutaminic acids is from 1:2 to 40, and also the molar ratio of (nucleic acid-based taste-active component + glutaminic acids):potassium: (lactic acid, sodium lactate or potassium lactate) is 1:5 to 80:10 to 80.

Also, Patent Literature 8 describes a yeast which has resistance against a glutamic acid-antagonizing growth inhibitor and accumulates glutaminic acid in cells.

Also, Patent Literature 9 describes a method for producing a yeast extract, the method includes using a Yarrowia Lipolytica yeast which has resistance against a drug nystatin which affects a structure and function of a cell membrane, and also has the ability of accumulating 530 mg/l or more of L-glutaminic acid in cells.

[Citation List]

[Patent Literature]

[Patent literature 1] Japanese Unexamined Patent Application, First Publication No. 2007-49989
[Patent literature 2] Japanese Patent No. 3,519,572
[Patent literature 3] Japanese Patent No. 3,088,709
[Patent literature 4] Japanese Unexamined Patent Application, First Publication No. 2002-171961
[Patent literature 5] Japanese Unexamined Patent Application, First Publication No. 2005-102549

[Patent literature 6] Japanese Unexamined Patent Application, First Publication No. 2006-129835
[Patent literature 7] Japanese Unexamined Patent Application, First Publication No. Hei 5-227911
[Patent literature 8] Japanese Unexamined Patent Application, First Publication No. Hei 9-294581
[Patent literature 9] Japanese Patent No. 3,896,606

[Summary of the Invention]

[Technical Problem to be Solved]

However, in conventional methods, in order to include a sufficient amount of a free amino acid in the case of producing various extracts using a vegetable or animal protein, operation becomes complicated in many cases, for example, it is necessary to carry out decomposition treatments as in hydrolyzed vegetable protein (HVP) hydrolyzed animal protein (HAP) or the like. In addition, there has been required a method for producing a yeast extract containing an amino acid or the like in a higher concentration than that of a yeast extract which is currently commercially available.
Regarding Patent Literature 1, in addition to a complicated operation in which an enzyme is used, the amount of glutaminic acid per dry powder is about 13%.
Also, regarding Patent Literature 2, in addition to a complicated operation in which a gene mutation treatment is carried out and an enzyme is used, and the safety, preference and the like of foods is inferior.
Also, Patent Literature 3 describes a yeast extract containing 1 to 20% of sodium glutamate. However, a commercially available product containing 5.0% of sodium glutamate is actually used and there is no mention of others.
Also, regarding Patent Literature 4, the production is carried out by genetic recombination and the operation is complicated, and the safety, preference and the like of foods is inferior.
Also, Patent Literature 5 describes that sodium (soda) glutamate is contained in a proportion of 10% or more per solid parts and there is no mention of it in the Examples.
Also, regarding Patent Literature 6, an operation is complicated, for example, and enzymegenation is carried out.
Also, regarding Patent Literature 7, glutaminic acidis are exernally added, merely.
Also, regarding Patent Literature 8, the amount of glutaminic acid on a dry weight basis of cells is low.
Also, regarding Patent Literature 9, an operation is complicated, and, for example, chemical resistance is imparted to parent strains.
Under these circumstances, the present invention has been made and an object of the present invention is to provide a method for producing an amino acid-rich yeast containing amino acid at a higher concentration than that of a conventional yeast.

[Solution to Solve the Problem]

The present inventors have intensively studied so as to achieve the above object and found that the amount of amino acid in a yeast increases by increasing the pH of culture fluid to a specific pH (shifting to an alkaline region) during culturing of the yeast in a stationary phase. They have also found that a yeast extract having a high amount of amino acid can be produced by producing a yeast extract using this yeast, and thus the present invention has been completed. The present invention relates to the following:

[1] A method for producing an amino acid-rich yeast, comprising:

- liquid-culturing the yeast in a logarithmic proliferation phase at a temperature of 20 to 40°C and a pH of 3.5 to 7.5, and
- liquid-culturing a yeast in a stationary phase of proliferation under the condition that the pH of a liquid medium is equal to or higher than 7.5 and lower than 11.

[2] The method for producing an amino acid-rich yeast according to [1], wherein the liquid-culturing comprises: adjusting the pH of a liquid medium of the yeast in a stationary phase of proliferation to equal to or higher than 7.5 and lower than 11, and further culturing the yeast within the pH range.
[3] The method for producing an amino acid-rich yeast according to [1] or [2], wherein the yeast is Saccharomyces cerevisiae or Candida utilis.
[4] The invention also relates to an amino acid-rich yeast obtained by the method for producing an amino acid-rich yeast according to any one of [1] to [3].
[5] The disclosure describes the amino acid-rich yeast according to [4], wherein the amount of a free amino acid is from 7.5 to 18.0% by weight per dry yeast cell.
[6] The disclosure describes an amino acid-rich yeast extract extracted from the amino acid-rich yeast according to [5].

[7] The disclosure describes the amino acid-rich yeast extract according to [6], wherein the amount of a free amino acid derived from the yeast is from 50 to 70% by weight on a dry weight basis.

[8] The disclosure describes a seasoning composition including the amino acid-rich yeast extract according to [6] or [7].

[9] The disclosure describes an amino acid-containing food and drink, including the amino acid-rich yeast according to [4] or [5], the amino acid-rich yeast extract according to [6] or [7] or the seasoning composition according to [8].

[Advantageous Effects of Invention]

According to the method for producing an amino acid-rich yeast of the present invention, an amino acid-rich yeast having a remarkably increased amount of free amino acid can be produced in a simple and easy manner only by shifting the pH of a liquid medium of the yeast in a stationary phase to an alkali.

By performing an operation of extraction from the amino acid-rich yeast, an amino acid-rich yeast extract containing free amino acid in a high concentration is obtained.

[Brief Description of the Drawings]

Fig 1 is a graph showing a curve of the increase in a cell count versus the culture time in Example 1.

Fig. 2 is a graph showing a curve of the increase in weight of dry yeast cells versus the culture time in Example 1.

Fig. 3 is a graph showing a change in the pH of a liquid medium versus the culture time in Example 1.

[Description of Embodiments]

The method for producing an amino acid-rich yeast of the present invention includes liquid-culturing the yeast in a logarithmic proliferation phase at a temperature of 20 to 40°C and a pH of 3.5 to 7.5, and liquid-culturing a yeast in a stationary phase of proliferation under the condition that the pH of a liquid medium is equal to or higher than 7.5 and lower than 11.

Embodiments of the present invention will be described in detail below.

The yeast may be unicellular fungi and specific examples thereof include fungi of the genus Saccharomyces, fungi of the genus Shizosaccharomyces, fungi of the genus Pichia, fungi of the genus Candida, fungi of the genus Kluyveromyces, fungi of the genus Williopsis, fungi of the genus Debaryomyces, fungi of the genus Galactomyces, fungi of the genus Torulaspora, fungi of the genus Rhodotorula, fungi of the genus Yarrowia, fungi of the genus Zygosaccharomyces and the like.

Among these yeasts, from the viewpoint of edibility, Candida tropicalis, Candida lypolitica, Candida utilis, Candida sake, Saccharomyces cerevisiae and the like are preferable, and commonly used Saccharomyces cerevisiae and Candida utilis are more preferable.

In order to carry out the present invention, after the yeast is cultured in a liquid medium containing a carbon source, a nitrogen source, an inorganic salt or the like until a stationary phase, the yeast may be liquid-cultured under the condition that the pH of a liquid medium of the yeast in a stationary phase of proliferation is equal to or higher than 7.5 and lower than 11.

A medium composition of these bacterial strains is not particularly limited, and compositions which are utilized in a conventional method can be used. For example, as the carbon source, one, or two or more kinds selected from the group consisting of glucose, sucrose, acetic acid, ethanol, molasses, a sulfite pulp waste solution and the like, which are utilized in conventional culturing of microorganisms, are used. A the nitrogen source, one, or two or more kinds selected from the group consisting of urea, ammonia, inorganic salts such as ammonium sulfate, ammonium chloride or ammonium phosphate, and nitrogen-containing organic substances such as corn steep liquor (CSL), casein, yeast extract or peptone, are used. Furthermore, a phosphate component, a potassium component, and a magnesium component may be added to the medium, and conventional industrial raw materials such as calcium superphosphate, ammonium phosphate, potassium chloride, potassium hydroxide, magnesium sulfate and magnesium hydrochloride may be used. In addition, inorganic salts such as zinc, copper, manganese and iron ions may be used. In addition, vitamins, nucleic acid-associated substances and the like may be added.

As a culturing form, any of batch culturing, semi-batch culturing and continuous culturing may be used. However, semi-batch culturing or continuous culturing is industrially employed.

The culturing condition before pH adjustment may be according to the general condition for culturing yeast and, for example, the temperature is from 20 to 40°C, and preferably from 25 to 35°C, and the pH is from 3.5 to 7.5, and particularly desirably from 4.0 to 6.0. In addition, the aerobic condition is preferred.

It is preferable to perform culturing while aeration and stirring. The amount of aeration and stirring condition can be appropriately determined taking account of the volume and the time of culturing, and the initial concentration of a fungus. For example, culturing can be performed while aeration of about 0.2 to 2 volume per volume per minute (V.V.M.) and stirring at about 50 to 800 rpm.

The method of liquid-culturing under the condition that the pH of a liquid medium of the yeast in a stationary phase of proliferation is equal to or higher than 7.5 and lower than 11 is not particularly limited. For example, the pH of a liquid medium may be adjusted to be equal to or higher than 7.5 and lower than 11 when yeast culturing has entered a stationary phase. Alternatively, a liquid medium may be alkali-shifted by adding urea to a medium in advance such that the pH naturally becomes equal to or higher than 7.5 and lower than 11 with a lapse of the culture time.

The amount of urea or the like to be added to the medium is not particularly limited, and is preferably from about 0.5 to 5% relative to the medium, although it depends on the cell concentration of the yeast to be cultured.

The method of adjusting the pH of a liquid medium to be equal to or higher than 7.5 and lower than 11 when the cultured yeast has entered a stationary phase is not particularly limited. For example, an alkaline component is appropriately added, and the pH of a liquid medium may be adjusted to be equal to or higher than 7.5 and lower than 11, preferably equal to or higher than 7.5 and lower than 10.

The adjustment of the pH may be performed as long as yeast is in a stationary phase, and it is preferable to perform pH adjustment immediately after yeast has entered a stationary phase. This is because not only the concentration of free amino acid in the yeast can be sufficiently enhanced, but also the required time until completion of all steps can be shortened.

When the pH of the liquid medium of the yeast in a logarithmic proliferation phase is adjusted to be equal to or higher than 7.5 and lower than 11, proliferation of the yeast is suppressed, and thus the free amino acid content is not increased, which is unfavorable.

Examples of the alkaline component include, but are not limited to, inorganic alkalis such as $NH_4OH$ (aqueous ammonia ), ammonia gas, sodium hydroxide, potassium hydroxide, calcium hydroxide, and magnesium hydroxide; alkaline bases such as sodium carbonate, and potassium carbonate; organic alkalis such as urea; and the like.

Among these components, aqueous ammonia, an ammonia gas and urea are preferable.

The temperature and other conditions when the yeast in a stationary phase is cultured in a liquid medium having a pH of equal to or higher than 7.5 and lower than 11 may be according to the common condition for culturing yeast, and for example, the temperature is from 20 to 40°C, and is preferably from 25 to 35°C.

There is a tendency that the amount of free amino acid in the yeast after the pH has shifted to be equal to or higher than 7.5 and lower than 11 increases with a lapse of a culture time and, after reaching a peak, the amount decreases. In addition, this depends on the conditions such as the cell concentration of the yeast to be cultured, the pH and the temperature. It is surmised that when excessively cultured for a long term under the alkaline condition, the influence of an alkali on the yeast becomes too great. Therefore, in the present invention, an optimal culture time can be appropriately selected for every culturing condition, particularly, for every pH after alkali shift. By preparing a yeast extract using the yeast at a peak, a free amino acid-rich yeast extract having a very high amount of amino acid is obtained.

That is, by completing the culturing at a peak and recovering the yeast, an amino acid-rich yeast extract having a very high amount of free amino acid of 7.5% by weight or more per dry yeast cell can be obtained. Also, by preparing a yeast extract using the yeast at a peak, an amino acid-rich yeast extract having a very high amount of free amino acid of 30% by weight or more on a dry weight basis can be obtained.

In the present invention, "the amount of free amino acid per dry yeast cell" means a ratio (% by weight) of free amino acid contained in solid parts obtained by drying the yeast cells. Also, "the amount of free amino acid on a dry weight basis of a yeast extract" means a ratio (% by weight) of free amino acid contained in solid parts obtained by drying the yeast extract.

As the method of measuring the amount of free amino acid in yeast cells or a yeast extract, for example, the amount can be measured by the AccQ-Tag Ultra labeling method using the Acquity UPLC analyzing apparatus manufactured by Waters (USA). A calibration curve may be made, for example, using an amino acid mixed standard solution H type (manufactured by Wako Pure Chemical Industries, Ltd.). It is possible to selectively determine the quantity of free amino acid in the sample by the method.

The amount can also be measured using an amino acid automatic analyzing apparatus manufactured by JEOL Ltd., Model JLC-500/V, but the method is not particularly limited.

Specifically, the amount of free amino acid of the yeast gradually increase with a lapse of the time as the culture time after alkali shift (after adjusting the pH of a liquid medium to the range between 7.5 and 11) increases. After reaching a peak at the time of the culture time of 6 to 12 hours, even if culturing is continued for about 48 hours, there is a tendency that the amount of free amino acid higher than that before alkali shift is maintained. Therefore, in order to obtain a yeast having a high amount of free amino acid, the culture time after alkali shift is preferably within 48 hours, more preferably 12 hours, and still more preferably 1 to 6 hours, after the pH adjustment.

As described above, according to the method of the present invention, a yeast having a very high amount of free amino acid can be produced. Also, by producing a yeast extract through extraction from the obtained yeast, a yeast extract containing rich free amino acid as a satisfactory taste-active component can be obtained in a simple and easy manner.

The present invention can produce amino acid-rich yeast by a simple step of only performing alkali shift of liquid medium. As described above, it is not necessary to use a particularly special medium, but the yeast can be produced using an inexpensive raw material such as ammonia.

According to the method of the present invention, an amino acid-rich yeast containing free amino acid in a high concentration in yeast cells is obtained. However, a fraction containing amino acid may be obtained from the amino acid-rich yeast.

As the method of fractionating a fraction containing amino acid from the amino acid-rich yeast, any method may be used as long as it is a method which is usually performed.

An amino acid-rich yeast extract can be produced from the amino acid-rich yeast cultured by the above method. As a method of producing the amino acid-rich yeast extract, any method may be used as long as it is a method which is usually performed, and a self-digesting method, an enzyme degrading method, an acid degrading method, an alkali extracting method, a hot water extracting method and the like are employed. Commonly, it is considered that amino acid in a yeast extract obtained only by the hot water extracting method is free amino acid in an approximately all amount, unlike the yeast extract obtained by an enzyme reacting method such as the self-digesting method.

The amino acid-rich yeast contains a large amount of free amino acid, and can contain free amino acid in a concentration of 7.5% by weight or more, and preferably 7.5% to 18.0% by weight, per dry yeast cell. Therefore, even when a yeast extract is simply extracted only by a hot water treatment, a yeast extract having satisfactory taste is obtained.

Heretofore, in order to enhance the amount of a taste-active amino acid such as free glutamic acid, a hydrolysis treatment using an acid or an alkali has been commonly performed using a vegetable or animal protein. However, a hydrolysate of a protein has a problem that it contains MCP (chloropropanols) which is suspected to be carcinogenic.

To the contrary, since the amino acid-rich yeast produced by the method of the present invention has originally a high free amino acid content, a yeast extract having a sufficiently high amount of free amino acid can be prepared without a decomposition treatment with an acid or an alkali, or enzyme treatment, after the yeast is extracted by the hot water method or the like. That is, by using the amino acid-rich yeast, a yeast extract excellent in both of taste-active properties and safety can be produced in a simple and easy manner.

The thus obtained amino acid-rich yeast extract contains a yeast cell-derived free amino acid in a concentration of 30% by weight or more, and preferably 30 to 70% by weight, on a dry weight basis in the yeast extract.

Therefore, the yeast extract obtained by the present invention has very high taste-active property, and a food and drink having deep taste and richness can be produced by using it in a food and drink.

Furthermore, by formulating the amino acid-rich yeast extract into powders, an amino acid-rich yeast extract powder is obtained and by appropriately selecting a yeast fungus, a yeast extract powder containing free amino acid of 30% by weight or more is obtained.

Dry yeast cells may be prepared from the amino acid-rich yeast cultured by the above method. As the method of preparing the dry yeast cell, any method may be used as long as it is a conventional method. However, a lyophilization method, a spray drying method, a drum drying method and the like are industrially employed.

The amino acid-rich yeast, dry yeast cells of the yeast, a yeast extract prepared from the yeast, and the yeast extract powder may be formulated into a seasoning composition. The seasoning composition may consist only of the yeast extract, or may contain other components such as a stabilizer, a preservative, in addition to the yeast extract or the like. The seasoning composition can be appropriately used in various foods and drinks, similar to other seasoning compositions.

Furthermore, the disclosure describes food and drink containing the amino acid-rich yeast obtained by the above method, or the amino acid-rich yeast extract extracted from the amino acid-rich yeast. By including the amino acid-rich yeast or the like, foods and drinks containing free amino acid in a high concentration can be effectively produced.

Usually, these foods and drinks may be any foods and drinks as long as they are foods and drinks to which a dry yeast, a yeast extract, and a seasoning composition containing them can be added, and examples thereof include alcoholic drinks, refreshing drinks, fermented foods, seasonings, soups, breads, confectionaries and the like.

In order to produce the foods and drinks, a preparation obtained from the above amino acid-rich yeast, or a fraction of the amino acid-rich yeast may be added in the production process of foods and drinks. In addition, as a raw material, the amino acid-rich yeast may be used as it is.

The present invention will be described in more detail by way of Examples, but the present invention is not limited to the following Examples.

Example 1

Saccharomyces cerevisiae AB9813 strain was cultured, and extract extraction from a yeast culture fluid, and free amino acid analysis were performed, by the methods shown in the following <1> to <8>.

<1> Pre-culturing

Two media consisting of the following composition were prepared (volume of 350 mL, 2 L baffled Erlenmeyer flask).

(Medium composition)

| | |
|---|---|
| Molasses | 8% |
| Urea | 0.6% |

$(NH_4)_2SO_4$, 0.16% (Ammonium sulfate) and
$(NH_4)_2HPO_4$ 0.08% (Diammonium hydrogen phosphate)

(Preparation method)

(1) Molasses (sugar content 36%, 167 ml) was diluted to 750ml with milli-Q water, and then each 350 ml was dispensed into a 2 L baffled Erlenmeyer flask.
(2) Autoclaving (121°C, 15 min) was performed.
(3) Upon use, a 1/50 amount (each 7 mL) of a nitrogen component mixed solution (×100) was added sterilely to a medium of only molasses.

(Culturing conditions)

| | |
|---|---|
| Culture temperature | 30°C |
| Shaking | 160 rpm (rotary) |
| Culture time | 24 hours |

(Inoculating fungus amount: 300 mL)

<2> Main culturing

A medium consisting of following composition was prepared at a volume of 2,000 mL (set at 3 L at completion of semi-batching).

(Medium composition)

Ammonium chloride 0.18% (in terms of 3 L at completion of semi-batching) 5.3 g
$(NH_4)_2HPO_4$ 0.04% (Diammonium hydrogen phosphate, in terms of completion of semi-batching) 1.2 g

Subsequently, culturing was performed under the following conditions.

(Culturing condition)

| | |
|---|---|
| Culture temperature | 30°C |
| Aeration | 3 L/min |
| Stirring | 600 rpm |

pH control Lower limit control pH 5.0
(with 10% aqueous ammonia), no upper limit control
Anti-foaming agent Adecanate stock
Semi-batching medium Molasses (sugar degree 36%),
volume 800 mL (with 1 L Medium bottle, final 8%)

<3> pH shift

Then, immediately after the cultured yeast had entered a stationary phase, the pH of the culture fluid was shifted to an alkaline region (hereinafter referred to as pH shift) with an aqueous $NH_4OH$ solution (10%) (setting pH 9.0), followed by further culturing of the yeast. After 48 hours from initiation of main culturing, the culturing was completed.

<4> Cell collecting method

(1) The culture fluid in which yeast had been main-cultured was transferred to a 50 ml plastic centrifugation tube (Falcon 2070), and centrifugation (3,000 g, 25°C, 5 min, HP-26) was performed.
(2) The supernatant was discarded and pellets were suspended in 20 ml of milli-Q water, and then centrifugation (3,000 g, 20°C, 5 min, HP26) was performed. This operation was repeated twice.
(3) The supernatant was discarded, and pellets were suspended in 20 ml of milli-Q water.

<5> Measurement of weight of dry yeast cells

Into an aluminum dish (diameter 5 cm) which had been weighed in advance was taken 2 ml of a yeast suspension, and this was dried at 105°C for 4 hours.
A weight after drying (weight after drying yeast) was measured, and a weight of solid parts (weight of dry yeast cells, unit g/L) was calculated by the following equation (1).

$$\text{Weight of yeast after drying} - \text{weight of aluminum dish} = \text{weight of dry yeast cells} \qquad (1)$$

<6> Preparation of extract solution by hot water extracting method

(1) The remaining yeast suspension (about 18 ml) was centrifuged (3,000 g, 20°C, 5 min, HP-26).
(2) The remaining suspension (1.5 ml) was transferred to an Eppendorf tube, and the tube was transferred to a block heater and then heated at 80°C for 30 minutes (conversion into extract). Alternatively, it may be excessively heated in a warm bath at 100°C for 10 minutes (conversion into extract).
(3) Thereafter, the supernatant (extract solution) was separated by centrifugation (6,000 g, 4°C, 5 min).

<7> Method for measurement of the amount of free amino acid

The amount of free amino acid in the extract solution was measured. The amount of free amino acid was measured by the AccQ-Tag Ultra labeling method using the Acquity UPLC analyzing apparatus manufactured by Waters (USA). A calibration curve was made using an amino acid mixed standard solution (type H) (manufactured by Wako Pure Chemical Industries Ltd.). The measurement results are shown in Table 1. In Table 1, "total amino acid" means the amount of total free amino acid (sum total of the contents of the respective free amino acids) per weight of a dry yeast cell.

<8> Method for measurement of decomposition rate

The extract solution (500 μl) was taken into an aluminum dish and dried at 105°C for 4 hours. Thereafter, an extract weight ratio (w/w) was calculated from a dry weight before conversion into an extract.

Table 1

| | 30°C, Target pH 9, Before shift | 30°C, Target pH 9, 1 hour after shift | 30°C, Target pH 9, 2 hours after shift | 30°C, Target pH 9, 3 hours after shift | 30°C, Target pH 9, 6 hours after shift | 30°C, Target pH9, 48 hours after shift |
|---|---|---|---|---|---|---|
| Weight of dry yeast cells (g/L) | 87.3 | 83.2 | 122.3 | 85.2 | 87.5 | 83.9 |
| Total amino acid (% by weight in dry yeast cells) | 11.6 | 12.5 | 13.6 | 13.8 | 15.1 | 13.0 |

The amount of the total free amino acid in dry yeast cells gradually increased until at least 6 hours after culturing after alkali shift. Even 48 hours after culturing,an amount higher than that before alkali shift was maintained.

As is apparent from the above results, the amount of free amino acid in the yeast is increased by further culturing yeast through the adjustment of the pH to equal to or higher than 7.5 and less than 11 after a stationary phase. The amount of free amino acid increased after initiation of pH shift and the amount of free amino acid within 48 hours after pH adjustment was higher than the amount of free amino acid before pH shift in any case.

Example 2

Pre-culturing was carried out in the same manner as in Example 1 <1> and, thereafter, main culturing was carried out under the following conditions.

Without pH shift after a stationary phase, urea was added to a medium of main culturing in advance and an amino acid-rich yeast was obtained under the condition where the pH naturally shifts.

First, a medium consisting of following composition was prepared at a volume of 2,000 mL (set at 3 L at completion of semi-batching).

(Medium composition)

Ammonium chloride 0.18% (in terms of 3 L at completion of semi-batching) 5.3 g
$(NH_4)_2HPO_4$ 0.04% (diammonium hydrogen phosphate, in terms of completion of semi-batching) 1.2 g
Urea 1% (in terms of 3 L at completion of semi-batching) 30 g

Culturing was carried out except that the other conditions are the same as in Example 1. Fig. 1 is a graph showing a curve of the increase in cell count versus the culture time. Fig. 2 is a graph showing a curve of the increase in the weight of dry yeast cells versus the culture time. Fig. 3 is a graph showing the change in pH of a culture fluid versus the culture time.

As shown in Fig. 1, it was confirmed that the increase in cell count ($\times 10^6$ cells/ml) reached the stationary state after 18 hours from culturing, and yeast entered a stationary phase of proliferation. In addition, the weight of dry yeast cells (g/L) also became into the approximately stationary state after 24 hours from culturing, and a stationary phase of proliferation was confirmed. The pH of the culture fluid was measured and, as a result, as shown in Fig. 3, the pH was shifted to an alkali (7.5 or higher and lower than 11), after the yeast had entered a stationary phase of proliferation. The amount of the total free amino acid per weight of dry yeast cell and the amount of the total free amino acid on a dry weight basis of the yeast extract are shown in Table 2.

Table 2

| Target pH | | Weight of dry yeast cells (g/L) | Total amino acid (% by weight in dry yeast cells) | Total amino acid (% by weight in extract) |
|---|---|---|---|---|
| 7.0 | Before pH shift | 36.6 | 6.0 | 24.0 |
| | 6 hours after pH shift | 36.6 | 10.8 | 42.0 |
| 7.5 | Before pH shift | 33.9 | 7.6 | 30.0 |
| | 6 hours after pH shift | 35.2 | 11.3 | 41.0 |
| 8.0 | Before pH shift | 33.3 | 8.1 | 32.0 |
| | 6 hours after pH shift | 33.0 | 8.7 | 33.0 |
| 9.0 | Before pH shift | 34.4 | 7.1 | 28.0 |
| | 6 hours after pH shift | 36.7 | 12.8 | 54.0 |

As is apparent from the above result, the content of free amino acid in the yeast can be increased by adding urea to a medium of main culturing in advance and further culturing the yeast after a stationary phase under the condition where the pH naturally shifts.

Example 3

Then, regarding the amount of a yeast extract produced from a yeast (pH 9.0) prepared in the same manner as in Example 1 and commercially available yeast extracts (Comparative Examples 1 to 8), the amount of total free amino acid on a dry weight basis of the yeast extract was measured and compared. The amount (% by weight) of total free amino acid on a dry weight basis of each yeast extract are shown in Table 3. In Table 3, the "amount of total amino acid" means the total amount of free amino acid on a dry weight basis of each yeast extract.

| Samples | Example 3 | Comparative Example 1 | Comparative Example 2 | Comparative Example 3 |
|---|---|---|---|---|
| Amount in extract | | Product of company A | Product of company B | Product of company C |
| Total amino acid (% by weight) | 60.2 | 8.6 | 5.6 | 11.1 |

| Samples | Comparative Example 4 | Comparative Example 5 | Comparative Example 6 |
|---|---|---|---|
| Amount in extract | Product of company D | Product of company E | Product of company F |
| Total amino acid (% by weight) | 8.8 | 9.9 | 3.6 |

| Samples | Comparative Example 7 | Comparative Example 8 |
|---|---|---|
| Amount in extract | Product of company G | Product of company H |
| Total amino acid (% by weight) | 21.0 | 17.0 |

As is apparent from the results, the yeast extract contains a large amount of free amino acid.

Commercially available yeast extracts examined this time contained free amino acid of at most 21% by weight and were not yeast extracts containing free amino acid in a very high concentration of 60% by weight, like the yeast extract described herein. Therefore, it was suggested that yeast extract extracted from the yeast produced by the method of the present invention is preferable as a seasoning.

Example 4

Furthermore, a miso soup and a consomme soup were made using a yeast extract powder (derived from Saccharomyces cerevisiae, AB9813 strain, amino acid: 60.2& by weight) obtained by formulating, a yeast extract produced from the yeast prepared in the same manner as in Example 1 (pH 9.0), into a powder. The amount of the yeast extract blended with the miso soup or the consomme soup is 0.2%.

As Comparative Example, using Meast Powder N (manufactured by ASAHI FOOD and HEALTHCARE CO., LTD.) (amino acid: 35.9% by weight), a miso soup and a consomme soup were made similarly, sensory evaluation was performed by the following method.

(Evaluation method)

A comparative sensory test was performed using blind two points comparison by 10 professional panelists. As a 2 pair comparative test, t-test was performed.

(Evaluation criteria)

With respect to three items, for example, salty taste (salt reduction effect), deliciousness and richness, a five-rank evaluation was performed, assumed that a miso soup as a standard or a consomme soup as a standard is 0.

"Strong" = +2,
"Slightly strong" = +1,
"Neither" = 0
"Slightly weak" = -1,
"Weak" = -2.

The results of the miso soup are shown in Table 4, and the results of the consomme soup are shown in Table 5.

Table 4

| Samples | Items | Panels | | | | |
|---|---|---|---|---|---|---|
| | | A | B | C | D | E |
| Examples | Salty taste | -1 | 1 | 1 | -1 | -1 |
| | Deliciousness | 0 | 0 | 1 | 2 | -1 |
| | Richness | 0 | 2 | 0 | 1 | 0 |
| Meast Powder N (Comparative Example) | Salty taste | 0 | 0 | 1 | -2 | 0 |
| | Deliciousness | 1 | 0 | 0 | 1 | -1 |
| | Richness | 1 | 2 | 0 | 0 | -1 |

| Samples | Items | Panels | | | | | | |
|---|---|---|---|---|---|---|---|---|
| | | F | G | H | I | J | Ave-rage | Standard deviation |
| Examples | Salty taste | 1 | 0 | 0 | 0 | 1 | 0.10 | 0.88 |
| | Deliciousness | 0 | 1 | 1 | 1 | -1 | 0.40 | 0.97 |
| | Richness | 0 | 1 | 2 | 1 | 1 | 0.80 | 0.79 |
| Meast Powder N (Comparative Example) | Salty taste | -1 | 0 | 0 | -1 | 0 | -0.30 | 0.82 |
| | Deliciousness | -1 | 1 | 0 | -1 | 0 | 0.00 | 0.82 |
| | Richness | 0 | 0 | 0 | 0 | 0 | 0.20 | 0.79 |

t-test for paired data

| | |
|---|---|
| Salty taste | p = 0.11 |
| Deliciousness | p = 0.11 |
| Richness | p = 0.03* |

Table 5

| Samples | Items | Panels | | | | |
|---|---|---|---|---|---|---|
| | | A | B | C | D | E |
| Examples | Salty taste | 1 | 1 | 1 | 1 | 0 |
| | Deliciousness | 1 | 0 | 1 | 2 | 1 |
| | Richness | 1 | 0 | 0 | 2 | 1 |
| Meast Powder N (Comparative Example) | Salty taste | 0 | -1 | 0 | -1 | 1 |
| | Deliciousness | 1 | -1 | 0 | -1 | 0 |
| | Richness | 1 | -1 | 1 | 1 | 1 |

| Samples | Items | Panels | | | | | | |
|---|---|---|---|---|---|---|---|---|
| | | F | G | H | I | J | Ave-rage | Standard deviation |
| Examples | Salty taste | -1 | 1 | 0 | 0 | 1 | 0.50 | 0.71 |
| | Delicious-ness | -1 | 2 | 2 | 0 | 1 | 0.90 | 0.99 |
| | Richness | 0 | 0 | 0 | 0 | 1 | 0.50 | 0.71 |
| Meast Powder N (Comparative Example) | Salty taste | 1 | 0 | 0 | 1 | -1 | 0.00 | 0.82 |
| | Delicious-ness | 1 | 1 | 0 | 0 | -1 | 0.00 | 0.82 |
| | Richness | 1 | 0 | 0 | 1 | -1 | 0.40 | 0.84 |

t-test for paired data

| | |
|---|---|
| Salty taste | p = 0.15 |
| Deliciousness | p = 0.03* |
| Richness | p = 0.38 |

As is apparent from the results of Table 4, in the miso soup, there was a difference in an average of salty taste and deliciousness, and there was a significant difference in richness. From the results of Table 5, in the consomme soup, there was a difference in an average of salty taste and richness, and there was a significant difference in deliciousness. It is considered that this is because the yeast extract has a significantly higher amount of free amino acid as compared with a conventional yeast extract.

Example 5

In the same manner as in Example 1, except that yeast to be cultured is Saccharomyces cerevisiae ABS1 strain, a yeast was cultured, and extraction of an extract from a yeast culture fluid and amino acid analysis were performed. The measured values of the amount of amino acid before and after pH shift are shown in Table 5.

Example 6

In the same manner as in Example 1, except that yeast to be cultured is Saccharomyces cerevisiae ABS2 strain, a yeast was cultured, and extraction of an extract from an yeast culture fluid and amino acid analysis were performed. The measured values of the amount of amino acid before and after pH shift are shown in Table 6.

Example 7

In the same manner as in Example 1, except that yeast to be cultured is Saccharomyces cerevisiae ABS3 strain, a yeast was cultured, and extraction of an extract from a yeast culture fluid and amino acid analysis were performed. The measured values of the amount of amino acid before and after pH shift are shown in Table 6.

Example 8

In the same manner as in Example 1, except that yeast to be cultured is Saccharomyces cerevisiae ABS5 strain, a yeast was cultured, and extraction of an extract from a yeast culture fluid and amino acid analysis were performed. The measured values of the amount of amino acid before and after pH shift are shown in Table 6.

Example 9

In the same manner as in Example 1, except that yeast to be cultured was Camellia (bread yeast), a yeast was cultured, and extraction of an extract from a yeast culture fluid and amino acid analysis were performed. The measured values of the amount of amino acid before and after pH shift are shown in Table 6.

Example 10

In the same manner as in Example 1, except that yeast to be cultured was Candida utilis ABC1 strain, a yeast was cultured, and extraction of an extract from a yeast culture fluid and amino acid analysis were performed. The measured values of the amount of amino acid before and after pH shift are shown in Table 6.

Example 11

In the same manner as in Example 1, except that yeast to be cultured was Candida utilis ABC2 strain, a yeast was cultured, and extraction of an extract from a yeast culture fluid and amino acid analysis were performed. The measured values of the amount of amino acid before and after pH shift are shown in Table 6.

Example 12

In the same manner as in Example 1, except that yeast to be cultured was Candida utilis ABC3 strain, a yeast was cultured, and extraction of an extract from a yeast culture fluid and amino acid analysis were performed. The measured values of the amount of amino acid before and after pH shift are shown in Table 6.

Table 6

| Bacterial strains | Target pH | Before pH shift | After pH shift |
|---|---|---|---|
| | | Total amino acid (% by weight in extract) | Total amino acid (% by weight in extract) |
| Saccharomyces cerevisiae ABS1 | 8.0 | 33.5 | 36.8 |
| Saccharomyces cerevisiae ABS2 | 8.0 | 19.2 | 20.8 |
| Saccharomyces cerevisiae ABS3 | 8.0 | 9.98 | 35.3 |
| Saccharomyces cerevisiae ABS5 | 9.0 | - | 60.2 |
| Camellia (Baker's yeast) | 8.0 | 13.5 | 29 |
| Candida utilis ABC1 | 8.0 | 5.89 | 17.2 |
| Candida utilis ABC2 | 8.0 | 5.19 | 34.2 |
| Candida utilis ABC3 | 8.0 | 5.97 | 43.2 |

As is apparent from the results of Table 6, a phenomenon of increase in the amount of amino acid was confirmed by pH shift, also in other strains of Saccharomyces cerevisiae, and the yeast of other genus which are tested in Example 1.

Example 13

In the same as that of Example 1, except that yeast to be cultured was Saccharomyces cerevisiae ABS4 strain, and the set pH of a difference of pH shift was in units of 0.5 between 7.0 to 9.5, a yeast was cultured and extraction of an extract from yeast culture fluid and amino acid analysis were performed. The measured values of the amount of amino acid before and after pH shift are shown in Table 7.

Table 7

| Saccharomyces cerevisiae | Target pH | Before pH shift | After pH shift |
|---|---|---|---|
| | | Total amino acid (% by weight in extract) | Total amino acid (% by weight in extract) |
| ABS4 | 7.0 | 27.7 | 36.4 |
| ABS4 | 7.5 | 27.5 | 35.3 |
| ABS4 | 8.0 | 28.3 | 41.9 |
| ABS4 | 8.5 | 26.4 | 40.6 |
| ABS4 | 9.0 | 29.3 | 36.7 |
| ABS4 | 9.5 | 27.3 | 29 |

[Industrial Applicability]

According to the method for producing an amino acid-rich yeast of the present invention, a yeast containing free amino acid maintained in a high concentration in cells can be obtained and therefore the present invention can be utilized in the field of food such as production of a yeast extract.

## Claims

**1.** A method for producing an amino acid-rich yeast comprising the steps of:

liquid-culturing the yeast in a logarithmic proliferation phase at a temperature of 20 to 40°C and a pH of 3.5 to 7.5, and
liquid-culturing the yeast in a stationary phase of proliferation under the condition that a pH of a liquid medium is equal to or higher than 7.5, and lower than 11.

**2.** The method for producing an amino acid-rich yeast according to claim 1, wherein the step of liquid-culturing a yeast in a stationary phase comprises:

adjusting the pH of a liquid medium of the yeast in a stationary phase of proliferation to equal to or higher than 7.5 and lower than 11, and
culturing the yeast within the above pH range.

**3.** The method for producing an amino acid-rich yeast according to claim 1 or 2, wherein the yeast is *Saccharomyces cerevisiae* or *Candida utilis.*

## Patentansprüche

**1.** Verfahren zum Herstellen einer aminosäurereichen Hefe, umfassend die Schritte:

Flüssigkultivieren der Hefe in einer logarithmischen Proliferationsphase bei einer Temperatur von 20 bis 40°C und einem pH von 3,5 bis 7,5, und
Flüssigkultivieren der Hefe in einer stationären Phase der Proliferation unter der Bedingung, dass ein pH eines flüssigen Mediums gleich oder höher als 7,5 und niedriger als 11 ist.

**2.** Verfahren zum Herstellen einer aminosäurenreichen Hefe nach Anspruch 1, wobei der Schritt des Flüssigkultivierens einer Hefe in einer stationären Phase umfasst:

Einstellen des pH eines flüssigen Mediums der Hefe in einer stationären Phase der Proliferation auf gleich oder höher als 7,5 und niedriger als 11, und
Kultivieren der Hefe innerhalb des vorstehenden pH-Bereichs.

**3.** Verfahren zum Herstellen einer aminosäurenreichen Hefe nach Anspruch 1 oder 2, wobei die Hefe *Saccharomyces cerevisiae* oder *Candida utilis* ist.

## Revendications

**1.** Procédé de production d’une levure riche en acide aminé comprenant les étapes de :

culture dans un liquide de la levure dans une phase de prolifération logarithmique à une température de 20 à 40 °C et un pH de 3,5 à 7,5, et
culture dans un liquide de la levure dans une phase de prolifération stationnaire sous réserve que le pH d’un milieu liquide soit supérieur ou égal à 7,5 et inférieur à 11.

**2.** Procédé de production d’une levure riche en acide aminé selon la revendication 1, dans lequel l’étape de culture dans un liquide d’une levure dans une phase stationnaire comprend :

l’ajustement du pH d’un milieu liquide de la levure dans une phase de prolifération stationnaire à une valeur supérieure ou égale à 7,5 et inférieure à 11, et
la culture de la levure dans la plage de pH ci-dessus.

**3.** Procédé de production d’une levure riche en acide aminé selon la revendication 1 ou 2, dans lequel la levure est *Saccharomyces cerevisiae* ou *Candida utilis.*

FIG. 1

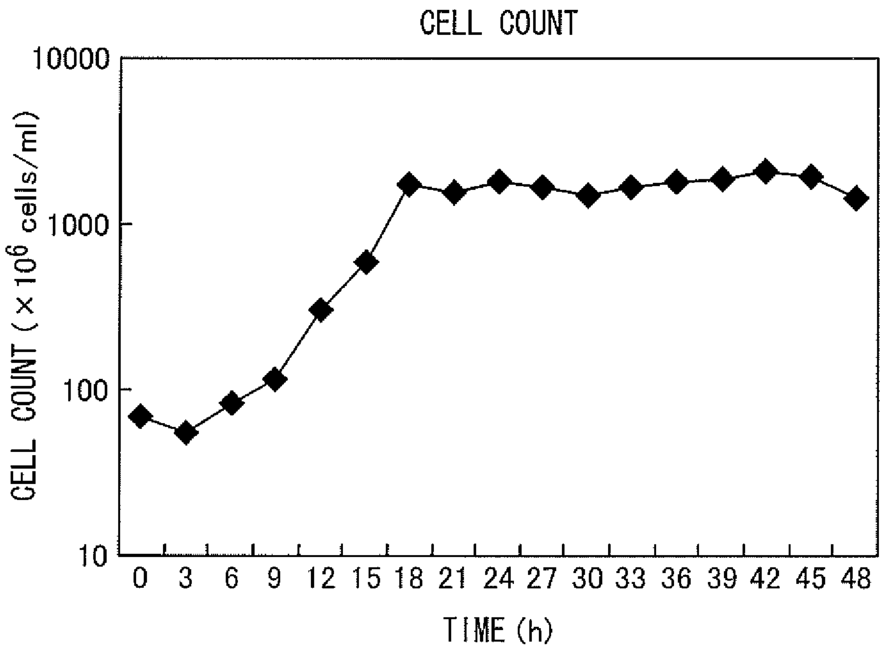
CELL COUNT
CELL COUNT (×10^6 cells/ml)
10000
1000
100
10
0 3 6 9 12 15 18 21 24 27 30 33 36 39 42 45 48
TIME (h)

FIG. 2

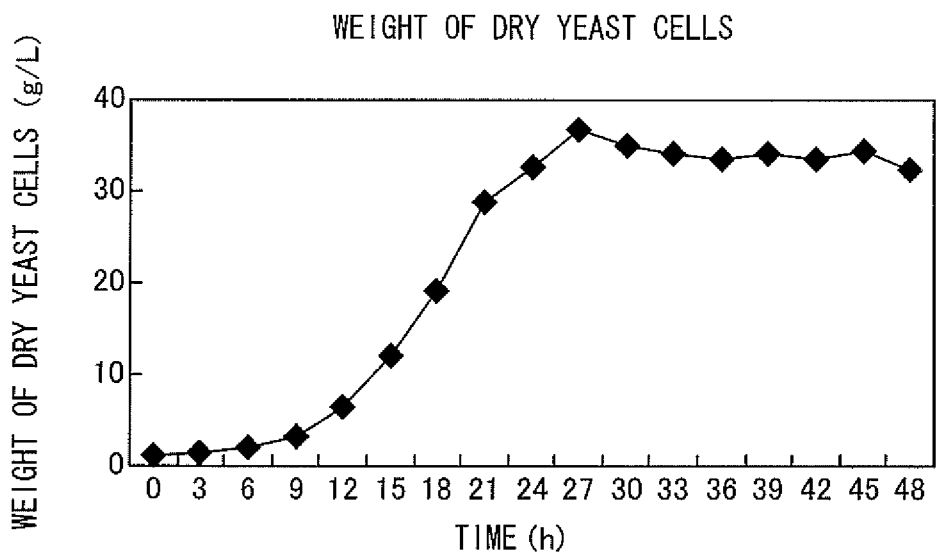
WEIGHT OF DRY YEAST CELLS
WEIGHT OF DRY YEAST CELLS (g/L)
40
30
20
10
0
0 3 6 9 12 15 18 21 24 27 30 33 36 39 42 45 48
TIME (h)

FIG. 3

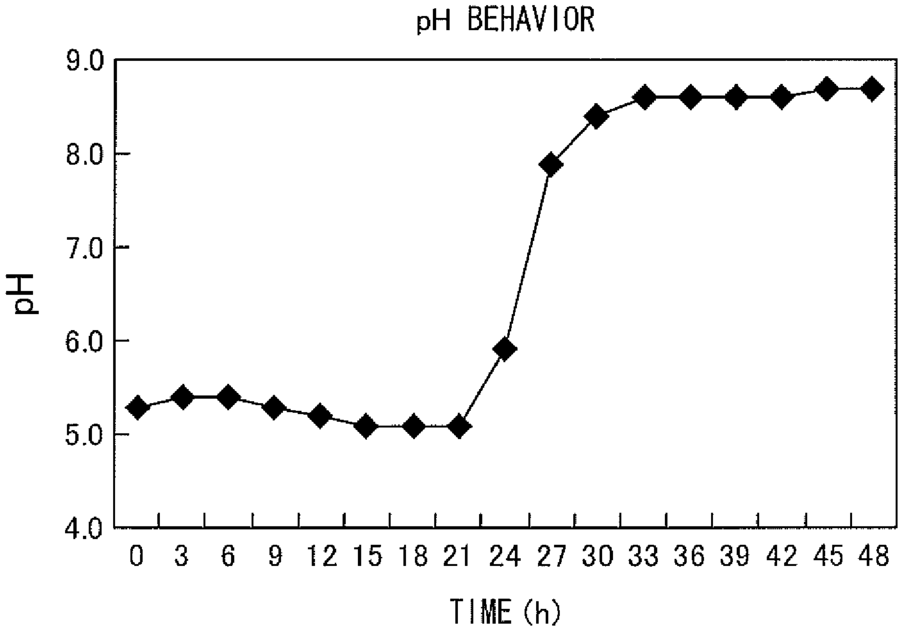
pH BEHAVIOR
9.0
8.0
7.0
6.0
5.0
4.0
pH
0 3 6 9 12 15 18 21 24 27 30 33 36 39 42 45 48
TIME (h)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007049989 A
- JP 3519572 B
- JP 3088709 B
- JP 2002171961 A
- JP 2005102549 A
- JP 2006129835 A
- JP HEI5227911 B
- JP HEI9294581 B
- JP 3896606 B